# EUROPÄISCHE PATENTSCHRIFT

(11) **EP 0 557 835 B1**
(45) Veröffentlichungstag und Bekanntmachung des Hinweises auf die Patenterteilung: **01.05.1996**
(21) Anmeldenummer: 93102296.6
(22) Anmeldetag: 13.02.1993
(51) Int. Cl.: C07C 231/12, C07C 233/36

(54) **Verfahren zur Herstellung von Betainen**
Process for the preparation of betaines
Procédé pour la préparation de bétaines

(30) Priorität: 26.02.1992 DE 4205880
(43) Veröffentlichungstag der Anmeldung: 01.09.1993
(73) Patentinhaber: Th. Goldschmidt AG, D-45127 Essen (DE)
(72) Erfinder: Grüning, Burghard, Dr., W-4300 Essen 1 (DE); Weitemeyer, Christian, Dr., W-4300 Essen 1 (DE); Foitzik, Willi, W-4250 Bottrop (DE); Käseborn, Hans-Dieter, W-4300 Essen 1 (DE)

(56) Entgegenhaltungen:
- EP-A- 0 020 907
- EP-A- 0 192 145
- EP-A- 0 302 329
- WO-A-91/08193
- FR-A- 2 348 190

## Beschreibung

Die Erfindung betrifft ein Verfahren zur Herstellung von Betainen der allgemeinen Formel 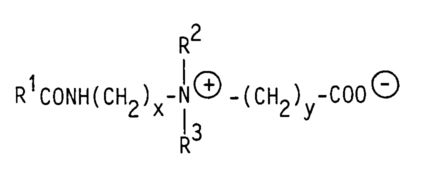
wobei
- R¹CO: ein von einer Fettsäure oder einem Fettsäuregemisch mit 6 bis 18 Kohlenstoffatomen abgeleiteter Acylrest ist,
- R² und R³: gleich oder verschieden sind und Alkylreste mit 1 bis 4 Kohlenstoffatomen bedeuten,
- x: = 2 oder 3 und
- y: = 1, 2 oder 3 ist,
durch Quaternierung der Fettsäureamiddialkylamine der allgemeinen Formel 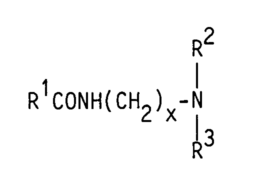
mit ω-Halogenalkylcarbonsäuren der Formel X-(CH₂)_{y}COOH, wobei X ein Halogenrest ist, oder deren Salzen in wäßriger Lösung.

Die Erfindung betrifft insbesondere ein Verfahren, welches die Herstellung von Betainen ermöglicht, die frei von organisch gebundenem Chlor und insbesondere frei von Natriummono- und -dichloracetat sind.

Als Carbonsäuren R¹COOH können insbesondere gesättigte und ungesättigte Fettsäuren eingesetzt werden, wobei auch Gemische von Fettsäuren, insbesondere Cocosfettsäure und Palmkernfettsäure, verwendet werden können.

Betaine werden in großem Umfang zur Herstellung von Körperreinigungsmitteln, insbesondere zur Herstellung von Haarwaschmitteln verwendet. Man ist deshalb bestrebt, die Betaine frei von Verunreinigungen herzustellen, welche Hautreizungen verursachen können oder in sonstiger Weise aus physiologischen Gründen unerwünscht sind.

In der DE-PS 29 26 479 ist ein Verfahren der obenbezeichneten Gattung beschrieben, welches dadurch gekennzeichnet ist, daß man die Quaternierungsreaktion während des gesamten Reaktionsablaufes in alkalischer Lösung, die bei 98°C gemessen einen pH-Wert von 7,5 bis 10,5 aufweist, durchführt.

Durch die Einhaltung des pH-Wertes bei der Quaternierungsreaktion wird erreicht, daß sich nach einer Reaktionszeit von etwa drei Stunden dünnschichtchromatographisch kein Fettsäureamiddialkylamin mehr nachweisen läßt, wobei die Nachweisgrenze bei etwa 0,02 Gew.-% liegt. Verlängert man die Reaktionszeit auf etwa 8 bis 10 Stunden, verringert sich auch der Gehalt an organisch gebundenem Chlor im Umsetzungsprodukt. Mit Verfeinerung der Analysenmethoden hat sich jedoch gezeigt, daß auch unter diesen Bedingungen im erhaltenen Betain noch Restmengen an Verbindungen mit organisch gebundenem Chlor, insbesondere Natriumdichloracetat enthalten sind. Die Anwendung höherer pH-Werte als 10,5 führt auch nur in unzureichendem Maße zu einer Verringerung der Restbestandteile an Chloressigsäuren. Hierzu wären übermäßig lange Reaktionszeiten notwendig, die sich aus wirtschaftlichen Gründen verbieten. Darüber hinaus besteht die Gefahr einer zunehmenden Zersetzung der Ausgangsprodukte bzw. der entstehenden Betaine, u.a. verursacht durch Hydrolyse der Amidbindung. Oberhalb eines pH-Wertes von 10,5 ist auch mit einer Zersetzung des Produktes zu rechnen.

Die DE-OS 39 39 264 betrifft ein Verfahren zur Erniedrigung des Restgehaltes an freiem Alkylierungsmittel in wäßrigen Lösungen amphoterer oder zwitterionischer Tenside, mit dem Kennzeichen, daß man die Lösungen mit Ammoniak, einer Aminosäure mit 2 bis 8 Kohlenstoffatomen oder einem Oligopeptid nachbehandelt. Durch diese Nachbehandlung soll der Restgehalt an freiem Alkylierungsmittel, insbesondere an Chloressigsäure, auf Werte unterhalb von 0,01 Gew.-% (bezogen auf Feststoffgehalt) gesenkt werden. Ein wesentlicher Nachteil dieses Verfahrens besteht jedoch darin, daß ein zusätzlicher Verfahrensschritt benötigt wird. Ein weiterer Nachteil ist darin zu erkennen, daß die Umsetzungsprodukte des Alkylierungsmittels mit Ammoniak, einer Aminosäure oder einem Oligopeptid im Verfahrensprodukt als Verunreinigung verbleiben.

EP-A-0 192 145 beschreibt die Quaternierung von einer Fettsäureamiddialkylamine mit Chloralkansäureestern in einem organischen Lösemittel durch Erhitzen beim Siedepunk des Lösemittels, insbesondere zwischen 60 und 180°C. Die Reaktion wird im allgemein so lange (1 bis 36 h) durchgeführt, bis das gebundene Chlor möglichst vollständig in Chloridionen überführt worden ist.

Die vorliegende Erfindung befaßt sich mit dem technischen Problem der Herstellung eines Betains, welches frei von Verunreinigungen, insbesondere frei von Verbindungen mit organisch gebundenem Chlor, wie Natriummono- und -dichloracetat, ist. Dabei soll nach Möglichkeit ein separater zusätzlicher Verfahrensschritt vermieden werden.

Erfindungsgemäß gelingt dies überraschenderweise dadurch, daß man die Reaktion innerhalb eines Temperaturbereiches von 115 bis 180°C durchführt, bis kein organisch gebundenes Chlor mehr nachweisbar ist, wobei man gegebenenfalls in einer ersten Stufe bei 80 bis 100°C das Fettsäureamiddialkylamin partiell oder vollständig quaterniert.

Vorzugsweise führt man die Reaktion bei einer Temperatur von 120 bis 160°C, insbesondere bei einer Temperatur von 120 bis 140°C durch. Die Reaktionsdauer, nach der kein organisch gebundenes Chlor mehr nachweisbar ist, beträgt in Abhängigkeit von der Temperatur etwa 1 bis 10 Stunden.

Es kann von Vorteil sein, zunächst in einer Vorreaktion bei 80 bis 100°C das Fettsäureamiddialkylamin partiell oder vollständig zu quaternieren und den Abbau des organisch gebundenen Chlors erst im Anschluß hieran bei der erhöhten Temperatur von mindestens 115°C vorzunehmen. Da bei der erhöhten Temperatur die Quaternierungsreaktion und die Abbaureaktion des organisch gebundenen Chlors konkurrierend verlaufen, muß bei der einstufigen Reaktionsweise eine überstöchiometrische Menge Halogenalkylcarbonsäure eingesetzt werden. Führt man jedoch die Quaternierungsreaktion bereits in einer ersten Stufe bei einer Temperatur von 80 bis 100°C partiell oder vollständig durch, ist es möglich, die Umsetzung mit stöchiometrischen Mengen oder mit einem nur geringen Überschuß an Halogenalkylcarbonsäure durchzuführen.

Es ist im Hinblick auf die gewählten Temperaturen erforderlich, in einem geschlossenen System, wie z.B. in einem entsprechend dimensionierten Rührautoklaven zu arbeiten.

Die Untergrenze des Temperaturbereiches von 115°C ist durch den Eintritt des Abbaus von Natriumdichloracetat gegeben. Unterhalb dieser Temperatur verläuft der Abbau nicht oder innerhalb eines Zeitraumes, der für ein wirtschaftlich durchzuführendes Verfahren nicht annehmbar ist. Die Obergrenze des Temperaturbereiches von 180°C ist durch die beginnende Zersetzung des Verfahrensproduktes bzw. der Reaktionspartner gegeben.

Mit dem erfindungsgemäßen Verfahren gelingt es, den Gehalt der Betainlösung an Natriummonochloracetat und Natriumdichloracetat unter die jeweilige Nachweisgrenze von < 10 ppm zu senken.

Das erfindungsgemäße Verfahren hat den Vorteil, daß dem Produkt zum Abbau der organischen Chlorverbindungen keine das Produkt verunreinigenden Reagenzien zugesetzt werden müssen.

In der DE-OS 27 23 120 ist zwar in einem Beispiel die Herstellung einer Verbindung der Formel 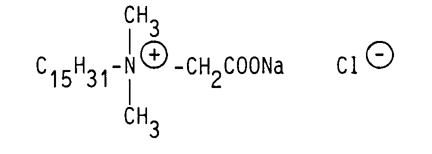
durch Umsetzung des entsprechenden Dimethylalkylamins bei 120 bis 130°C beschrieben. Da der Chlorgehalt dieses Produktes mit 11,5 % angegeben ist, wurde durch dieses Beispiel dem Fachmann nicht nahegelegt, diese Verfahrensweise auf Betaine zu übertragen, die von einem Fettsäureamiddialkylamin abgeleitet sind und bei denen überdies die Gefahr der hydrolytischen Spaltung der Amidbindung zu befürchten war.

In den folgenden Beispielen wird das erfindungsgemäße Verfahren im Vergleich zu dem Verfahren des Standes der Technik, wie er durch die DE-PS 29 26 479 gegeben ist, noch näher erläutert.

In den folgenden Beispielen wird als Fettsäureamid Cocosfettsäureaminoamid eingesetzt, welches durch Amidierung von Cocosfettsäure mit 3-N,N-Dimethylaminopropylamin hergestellt worden ist und einen durch Titration ermittelten Gehalt an tertiärem Stickstoff von 4,6 Gew.-% N aufweist. Die Titration erfolgt mit 0,1 M Salzsäure. Als Indikator wird Bromphenolblau verwendet. Als Natriummonochloracetat wird ein Produkt handelsüblicher Qualität eingesetzt, welches etwa 0,1 Gew.-% Natriumdichloracetat als Verunreinigung enthält.
1. Nichterfindungsgemäße Herstellung des Betains entsprechend DE-PS 29 26 479
1.1. 360 g Cocosfettsäureaminoamid werden zu einer Lösung von 128 g (1,1 Mol) Natriummonochloracetat in 828 g Wasser gegeben, mit 5,3 g 40 %iger wäßriger Natronlauge versetzt und im Dreihalskolben mit Rührer auf 98°C erhitzt. In regelmäßigen Abständen werden Proben genommen und hinsichtlich ihres Gehaltes an Natriummonochloracetat und Natriumdichloracetat mittels Kapillarelektrophorese untersucht.

| Nach 8 Stunden Reaktionszeit beträgt | |
|---|---|
| der Gehalt an Natriummonochloracetat | < 10 ppm und |
| der Gehalt an Natriumdichloracetat | 35 ppm. |

1.2. In einem Rührautoklaven werden 360 g Cocosfettsäureaminoamid zu einer Lösung von 139,7 g (1,2 Mol) Natriummonochloracetat in 828 g Wasser gegeben und mit 5,3 g 40 %iger wäßriger Natronlauge versetzt. Mit Stickstoff wird ein Druck von 7 bar eingestellt. Anschließend wird auf 100°C erhitzt. Es werden in regelmäßigen Abständen Proben genommen und auf ihren Gehalt an Natriummonochloracetat und Natriumdichloracetat untersucht. Die Reaktion wird nach 10 Stunden beendet. Das Produkt ist durch folgende Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | 115 ppm |
| Natriumdichloracetatgehalt: | 60 ppm |

Das Beispiel 1.2. zeigt, daß durch eine Druckerhöhung der Gehalt an organisch gebundenem Chlor im Betain nicht gesenkt werden kann. Der erhöhte Gehalt an Natriummonochloracetat und Natriumdichloracetat ist auf die Erhöhung des Zusatzes von Natriummonochloracetat und Natriumdichloracetat gegenüber Beispiel 1.1. zurückzuführen.
1.3. Es wird das Verfahren gemäß Beispiel 1.2. wiederholt, jedoch eine Reaktionstemperatur von 110°C gewählt, wobei sich ein Druck von ca. 1,5 bar ergibt. Die Reaktion wird nach 10 Stunden beendet. Das Produkt ist durch die folgenden Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | 35 ppm |

Das Beispiel 1.3. zeigt, daß bei einer Temperatur von 110°C und bei der Einhaltung der Stöchiometrie des Reaktionsansatzes von Beispiel 1.2. zwar der Gehalt an Natriummonochloracetat unter 10 ppm absinkt, der Gehalt an Natriumdichloracetat jedoch noch 35 ppm beträgt.
2. Erfindungsgemäße Herstellung des Betains
2.1. 360 g Cocosfettsäureaminoamid werden zu einer Lösung von 139,7 g (1,2 Mol) Natriummonochloracetat in 828 g Wasser gegeben und im Rührautoklaven auf 120°C erhitzt, wobei sich ein Druck von ca. 2 bar einstellt. In regelmäßigen Abständen werden Proben genommen. Die Reaktion wird nach 8 Stunden beendet. Das Produkt ist durch die folgenden Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | < 10 ppm |

2.2. Der gleiche Reaktionsansatz wie in Beispiel 2.1. wird im Rührautoklaven auf 140°C erhitzt, wobei sich ein Druck von ca. 3,5 bar einstellt. Die Reaktion wird nach 4 Stunden beendet. Das Produkt ist durch folgende Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | < 10 ppm |

2.3. Es wird das Verfahren des Beispiels 2.2. wiederholt. Es erfolgt jedoch keine Zugabe von Natronlauge. Die Reaktion wird nach 8 Stunden beendet. Das Produkt ist durch folgende Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | < 10 ppm |

2.4. Es wird das Verfahren des Beispiels 2.2. wiederholt, jedoch wird das Reaktionsgemisch im Rührautoklaven auf 160°C erhitzt, wobei sich ein Druck von ca. 4,5 bar ergibt. Bereits nach 1 Stunde Reaktionszeit wird der Natriumdichloracetatgehalt zu < 10 ppm bestimmt. Die Reaktion wird nach 4 Stunden beendet. Das Produkt ist durch folgende Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | < 10 ppm |

2.5. Es wird das Verfahren des Beispiels 2.3. wiederholt, jedoch wird das Reaktionsgemisch im Rührautoklaven auf 180°C erhitzt, wobei sich ein Druck von ca. 8 bar ergibt. Bereits nach 1 Stunde wird der Natriumdichloracetatgehalt zu < 10 ppm bestimmt. Die Reaktion wird nach 4 Stunden beendet. Im Produkt werden durch ¹H-NMR-Spektroskopie Zersetzungsprodukte in einer Menge von etwa 5 Gew.-% des Betaingehaltes festgestellt. Das Produkt ist durch folgende Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | < 10 ppm |

2.6. 360 g Cocosfettsäureaminoamid werden zu einer Lösung von 122,2 g (1,05 Mol) Natriummonochloracetat in 828 g Wasser gegeben. Die Reaktionsmischung wird auf 95°C erwärmt. Der pH-Wert wird durch kontinuierliche Zugabe 40 %iger Natronlauge bei 8,5 bis 9 gehalten. In regelmäßigen Abständen werden Proben entnommen und dünnschichtchromatographisch untersucht. Nachdem kein Cocosfettsäureaminoamid mehr nachweisbar ist, wird die Reaktionstemperatur auf 140°C erhöht und für 3 Stunden beibehalten. Die Reaktion ist anschließend beendet. Das Produkt ist durch folgende Daten charakterisiert:

| | |
|---|---|
| Festkörpergehalt: | 35 Gew.-% |
| Natriumchloridgehalt: | 5,5 Gew.-% |
| Cocosfettsäureaminoamidgehalt: | ca. 0,1 Gew.-% |
| Natriummonochloracetatgehalt: | < 10 ppm |
| Natriumdichloracetatgehalt: | < 10 ppm |

In dem Diagramm sind die Konzentrationen von Natriumdichloracetat in Betain bei einer Umsetzung bei 100°C gemäß Beispiel 1.2., bei 110°C gemäß Beispiel 1.3. sowie bei 120°C gemäß Beispiel 2.1., bei 140°C gemäß Beispiel 2.2. und bei 140°C gemäß Beispiel 2.3. dargestellt. Aus diesem Diagramm kann entnommen werden, daß die untere Temperatur des beim erfindungsgemäßen Verfahren einzuhaltenden Temperaturintervalls bei etwa 115°C liegt. Unterhalb dieser Temperatur erfolgt kein rascher Abfall des Gehaltes an Natriumdichloracetat unter 10 ppm. Gleichzeitig ergibt sich in Abhängigkeit von der Reaktionstemperatur eine Umsetzungszeit von etwa 2 bis 8 Stunden je nach gewählter Temperatur. Im Hinblick auf die Zersetzungsprodukte gemäß Beispiel 2.5. ist als Obergrenze des Temperaturbereichs für das erfindungsgemäße Verfahren eine Temperatur von 180°C anzunehmen, jedoch eine Obergrenze der Reaktionstemperatur von 160°C bevorzugt.

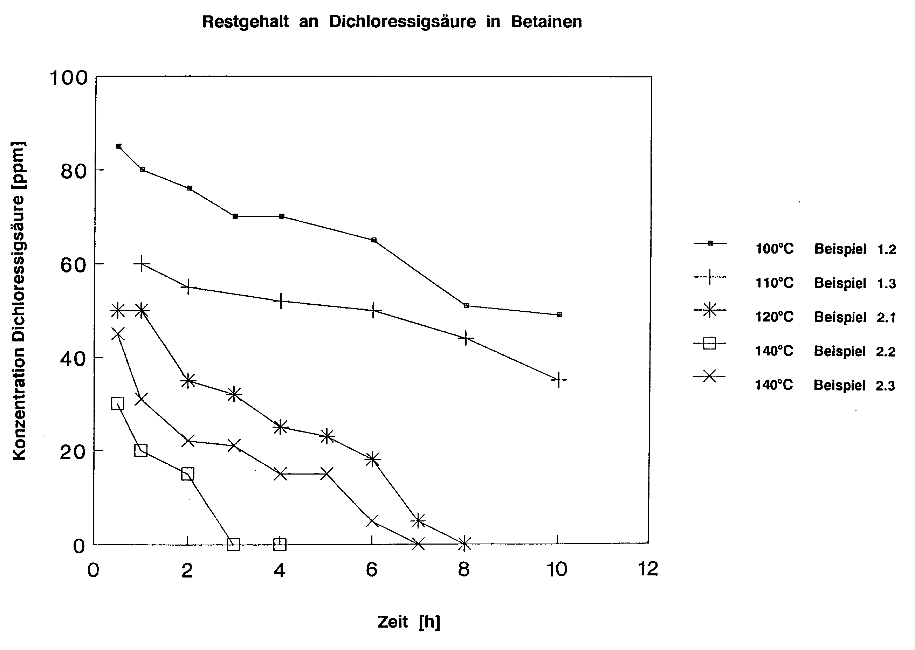

## Patentansprüche

1. Verfahren zur Herstellung von Betainen der allgemeinen Formel 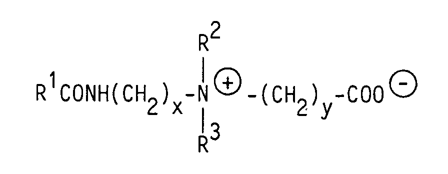 wobei
R¹CO ein von einer Fettsäure oder einem Fettsäuregemisch mit 6 bis 18 Kohlenstoffatomen abgeleiteter Acylrest ist,
R² und R³ gleich oder verschieden sind und Alkylreste mit 1 bis 4 Kohlenstoffatomen bedeuten,
x = 2 oder 3 und
y = 1, 2 oder 3 ist,
durch Quaternierung der Fettsäureamiddialkylamine der allgemeinen Formel 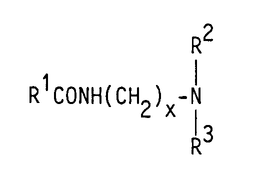 mit ω-Halogenalkylcarbonsäuren der Formel X-(CH₂)_{y}COOH, wobei X ein Halogenrest ist, oder deren Salzen in wäßriger Lösung, dadurch gekennzeichnet, daß man die Reaktion innerhalb eines Temperaturbereiches von 115 bis 180°C durchführt, bis kein organisch gebundenes Chlor mehr nachweisbar ist, wobei man gegebenenfalls in einer ersten Stufe bei 80 bis 100°C das Fettsäureamiddialkylamin partiell oder vollständig quaterniert.

2. Verfahren nach Anspruch 1, dadurch gekennzeichnet, daß man die Reaktion bei einer Temperatur von 120 bis 160°C durchführt.

3. Verfahren nach Anspruch 1, dadurch gekennzeichnet, daß man die Reaktion bei einer Temperatur von 120 bis 140°C durchführt.

## Claims

1. Process for the preparation of betaines of the general formula 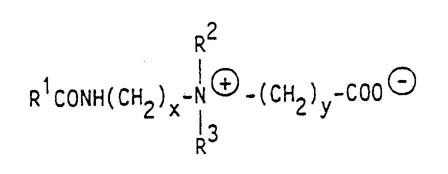 in which
R¹CO is an acyl radical derived from a fatty acid or a mixture of fatty acids having 6 to 18 carbon atoms,
R² and R³ are identical or different and denote alkyl radicals having 1 to 4 carbon atoms,
x = 2 or 3 and
y = 1, 2 or 3,
by quaternization of the fatty amidodialkylamines of the general formula 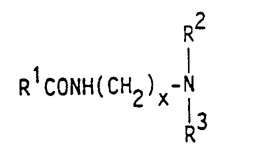 with ω-haloalkylcarboxylic acids of the formula X-(CH₂)_{y}COOH, where X is a halogen radical, or salts thereof in aqueous solution, characterized in that the reaction is carried out within a temperature range from 115 to 180°C until organically bound chlorine is no longer detectable, if appropriate the fatty amidodialkylamine being partially or completely quaternized in a first stage at 80 to 100°C.

2. Process according to Claim 1, characterized in that the reaction is carried out at a temperature of 120 to 160°C.

3. Process according to Claim 1, characterized in that the reaction is carried out at a temperature of 120 to 140°C.

## Revendications

1. Procédé de préparation de bétaïnes répondant à la formule générale : 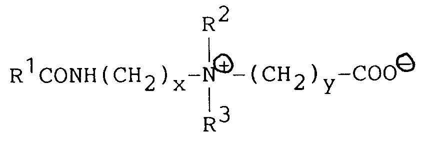 où
R¹CO est un reste acyle dérivé d'un acide gras ou d'un mélange d'acides gras ayant de 6 à 18 atomes de carbone,
R² et R³ sont identiques ou différents et signifient des restes alkyles ayant de 1 à 4 atomes de carbone,
x = 2 ou 3, et
y = 1, 2 ou 3,
par quaternisation des dialkylamines d'amides d'acides gras répondant à la formule générale : 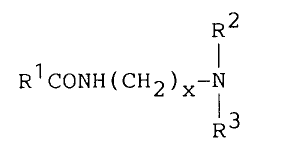 avec des acides ω-halogénoalkylcarboxyliques ayant la formule X-(CH₂)_{y}COOH, où X est un reste d'halogène, ou les sels de ceux-ci, en solution aqueuse,
caractérisé en ce qu'on effectue la réaction à une température comprise entre 115 et 180°C jusqu'à ce qu'on ne puisse plus déceler de chlore lié de manière organique, éventuellement en effectuant, lors d'une première étape, une quaternisation partielle ou totale de la dialkylamine d'amide d'acide gras à une température comprise entre 80 et 100°C.

2. Procédé selon la revendication 1, caractérisé en ce qu'on effectue la réaction à une température comprise entre 120 et 160°C.

3. Procédé selon la revendication 1, caractérisé en ce qu'on effectue la réaction à une température comprise entre 120 et 140°C.
